(19) Europäisches Patentamt / European Patent Office / Office européen des brevets

(11) **EP 4 641 493 A1**

(12) **EUROPEAN PATENT APPLICATION**
published in accordance with Art. 153(4) EPC

(43) Date of publication:
29.10.2025 Bulletin 2025/44

(21) Application number: 23907876.9

(22) Date of filing: 22.12.2023

(51) International Patent Classification (IPC):
*G06T 7/11* (2017.01)     *G06T 7/174* (2017.01)
*G06T 7/00* (2017.01)     *G06V 20/69* (2022.01)
*G06N 3/08* (2023.01)     *G01N 21/64* (2006.01)

(52) Cooperative Patent Classification (CPC):
G01N 21/64; G06N 3/08; G06T 7/00; G06T 7/11;
G06T 7/174; G06V 20/69

(86) International application number:
PCT/KR2023/021440

(87) International publication number:
WO 2024/136593 (27.06.2024 Gazette 2024/26)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 23.12.2022 KR 20220182527
20.04.2023 KR 20230052215

(71) Applicant: Korea Advanced Institute of Science
and Technology
Daejeon 34141 (KR)

(72) Inventors:
• CHANG, JaeByum
Daejeon 34141 (KR)
• SEO, Junyoung
Daejeon 34141 (KR)

(74) Representative: Keller Schneider
Patent- und Markenanwälte AG
Eigerstrasse 2
Postfach
3000 Bern 14 (CH)

(54) **IMAGE PROCESSING METHOD AND SYSTEM FOR REMOVING AUTOFLUORESCENCE AND CROSS-REACTIVITY OF ANTIBODIES USING SIGNAL SEPARATION ALGORITHM**

(57) Disclosed are an image processing method and system for removing autofluorescence and cross-reactivity of antibodies using a signal separation algorithm. An image processing method according to one embodiment may comprise the steps of: obtaining, from a biological tissue, a first unseparated image in which a first molecule is displayed and a second unseparated image in which the first molecule and a second molecule are simultaneously displayed; and generating a first separated image for the first molecule on the basis of the first unseparated image, and a second separated image for the second molecule on the basis of the first unseparated image and the second unseparated image.

FIG. 22

```
        ┌─────────┐
        │  Start  │
        └─────────┘
             │
             ▼
┌──────────────────────────────────────┐
│ Acquire, from biological tissue, first│
│ unseparated image in which first      │── 2210
│ molecule is displayed and second      │
│ unseparated image in which first      │
│ molecule and second molecule are      │
│ simultaneously displayed              │
└──────────────────────────────────────┘
             │
             ▼
┌──────────────────────────────────────┐
│ Generate first separated image for    │
│ first molecule based on first         │
│ unseparated image, and generate second│── 2220
│ separated image for second molecule   │
│ based on first unseparated image and  │
│ second unseparated image              │
└──────────────────────────────────────┘
             │
             ▼
        ┌─────────┐
        │   End   │
        └─────────┘
```

**Description**

TECHNICAL FIELD

The following description relates to an image processing method and system for removing autofluorescence and cross-reactivity of antibodies using a signal separation algorithm.

RELATED ART

Spatial proteomics has attracted considerable interest in its application to investigating the three-dimensional organization of cells and microenvironments of the cells in highly heterogeneous systems, such as tumors and the brain. This approach relies on microscopic imaging of proteins and immunofluorescence is a commonly employed method to accomplish this goal. The most widely used immunofluorescence method is an indirect immunostaining method that stains antigens with primary antibodies and then amplifies signals using fluorophore-conjugated secondary antibodies. However, when designing experiments for multiplexed imaging, several factors need to be considered to prevent signal cross talk between proteins. First, the use of fluorophores with similar emission spectra needs to be avoided. Second, primary antibodies from different host species need to be used. Third, when imaging specimens with high autofluorescence, the autofluorescence of specimens needs to be minimized using chemical or optical approach. To achieve multiplexed imaging with high specificity and accuracy, these three factors need to be carefully optimized and simultaneously considered. While cutting-edge spectral unmixing techniques may address the first issue, techniques for resolving the second and third issues have received relatively less attention.

The second issue, that is, the need to use primary bodies from different host species poses a significant challenge to the design of multiplexed imaging. In general, indirect staining achieves higher signal intensity than direct staining, which is desirable for imaging proteins in low abundance. In indirect staining, the use of primary antibodies from the same species may cause bleed-through between proteins, the phenomenon that signals from a plurality of proteins simultaneously appear in a single image, which requires the use of primary antibodies derived from different host species. However, most commercial primary antibodies are acquired from a limited number of fewer than 10 host species, usually rabbits or mice. This becomes increasingly problematic given recent advancement in spectrally unmixing techniques that enable the simultaneous use of four or more fluorophores and as many as 15 fluorophores. The use of secondary antibodies that may bind to specific isotypes or subclasses presents a potential salutation to this issue. Nevertheless, the availability of validated antibodies remains restricted to a small number of animal species, and commercially available isotypes or subclasses have limitation in constructing antibody combinations for detecting a plurality of proteins. An alternative solution to this issue involves using primary antibodies conjugated with oligonucleotides and signal amplification through DNA hybridization. However, a DNA-antibody conjugation procedure is expensive, time-consuming, labor-intensive, and complex, and needs to be optimized and validated for each antibody. **In** addition, DNA-conjugated primary antibodies may cause nonspecific binding, particularly, in cell nuclei. Therefore, there is a need for a method that enables the simultaneous use of primary antibodies derived from the same host species in indirect immunostaining to facilitate multiplexed imaging of proteins while maintaining a high detection limit.

The third issue, which is related to autofluorescence, also poses a considerable challenge to the design of multiplexed imaging. In particular, when imaging formalin-fixed paraffin-embedded (FFPE) specimens that exhibit high levels of autofluorescence, bleed-through in which autofluorescence signals mix into protein signals may occur, which may lead to significant misinterpretations in both quantitative and qualitative spatial proteomics analysis. Autofluorescence signals are distinctly observed across all visible light ranges due to the presence of various fluorescent molecules, such as nicotinamide adenine dinucleotide hydride, flavins, blood cells, collagen, elastin, lipofuscin, and paraffin. To eliminate autofluorescence, chemical quenching agents including Sudan black B and sodium borohydride have been tested on some organs. However, Sudan black B may reduce fluorescence signals from fluorophores and may introduce background signals, so may reduce a signal-to-noise ratio, while sodium borohydride may not completely eliminate autofluorescence signals. Since the distribution and expression of autofluorescence sources differ for each organ, chemical composition and concentration need to be optimized for each organ. Also, some chemical reagents may react with lipids, causing inappropriate labeling of proteins inserted into cell membranes. Another approach is to permanently eliminate autofluorescence by photochemically bleaching fluorescent molecules using light irradiation. However, a cooling system is required to prevent damage to antigens caused by heat generated by light irradiation. In particular, in the case of thick specimens, a photobleaching time may exceed one day. Photobleaching may also lead to the bleaching of fluorescent proteins' signals in transgenic model organisms, such as green fluorescent protein and tdTomato. Therefore, a method that may selectively eliminate autofluorescence signals without chemical or optical approach may be highly beneficial.

DETAILED DESCRIPTION

TECHNICAL SUBJECT

Example embodiments provide an image processing method and system for removing autofluorescence and cross-reactivity of antibodies using a signal separation algorithm.

SOLUTION

As an image processing method performed by a computer device including at least one processor and at least one memory in which instructions to be executed by the at least one processor are stored, provided is the image processing method including acquiring, from a biological tissue, a first unseparated image in which a first molecule is displayed and a second unseparated image in which the first molecule and a second molecule are simultaneously displayed; and generating a first separated image for the first molecule based on the first unseparated image, and generating a second separated image for the second molecule based on the first unseparated image and the second unseparated image.

According to an aspect, the acquiring may include acquiring the first unseparated image and the second unseparated image generated by irradiating light of different wavelengths to a specimen.

According to another aspect, the generating may include calculating the first unseparated image and the second unseparated image using an unmixing matrix.

According to still another aspect, a value of at least one element included in the unmixing matrix may be determined based on self-supervised learning.

According to still another aspect, a value of at least one element included in the unmixing matrix may be used to define a loss function corresponding to the first unseparated image and the second unseparated image, and the generating may include calculating variables that minimize the loss function; and generating the first separated image and the second separated image using the variables.

According to still another aspect, the loss function may be calculated using at least one of mutual information, Kullback-Leibler divergence, cross-entropy, and Rand-index.

According to still another aspect, the generating may include generating the first separated image and the second separated image using Gram-Schmidt (GS) orthogonalization.

According to still another aspect, the acquiring may include labeling the first molecule with a first material that contains a first fluorophore and labeling the first molecule and the second molecule with a second material that contains a second fluorophore and then, acquiring the first unseparated image that contains a signal of the first fluorophore and the second unseparated image that contains signals of the first fluorophore and the second fluorophore.

According to still another aspect, the first unseparated image may be acquired by detecting only light emitted from the first fluorophore, and the second unseparated image may be acquired by detecting only light emitted from the second fluorophore.

According to still another aspect, the first unseparated image and the second unseparated image may contain amplified fluorescence signals.

According to still another aspect, the acquiring may include labeling the first molecule with a first material that contains a first fluorophore, labeling the first molecule and the second molecule with a second material that contains a second fluorophore, and labeling the first molecule, the second molecule, and a third molecule with a third material that contains a third fluorophore and then, acquiring the first unseparated image that contains a signal of the first fluorophore, the second unseparated image that contains signals of the first fluorophore and the second fluorophore, and a third unseparated image that contains signals of the first fluorophore, the second fluorophore, and the third fluorophore, and the generating may include generating the first separated image for the first molecule based on the first unseparated image, generating the second separated image for the second molecule based on the first unseparated image and the second unseparated image, and generating a third separated image for the third molecule based on the second unseparated image and the third unseparated image.

According to still another aspect, the first molecule may include a molecule within the biological tissue that is not labeled with a fluorophore, and the second molecule may be labeled with a second material that contains a second fluorophore.

According to still another aspect, the first unseparated image may contain a self-luminous signal of the biological tissue itself.

According to still another aspect, the first unseparated image and the second unseparated image images acquired by irradiating light of the same wavelength to a specimen.

According to still another aspect, the first unseparated image may be an image acquired in a wavelength in which a signal of the second fluorophore is not detected.

According to still another aspect, the first unseparated image and the second unseparated image may be images acquired by irradiating light of different wavelengths to a specimen.

Provided is a computer program stored in a computer-readable recording medium to execute the method in conjunction with a computer device.

Provided is a computer-readable recording medium storing a program to execute the method on a computer device.

Provided is a computer device including at least one processor configured to execute computer-readable instructions on the computer device, and at least one memory configured to store the instructions to be executed by the at least one processor, wherein the at least one processor causes the computer device to acquire, from a biological tissue, a first unseparated image in which a first molecule is displayed and a second unseparated image in which the first molecule and a second molecule are simultaneously displayed, and to generate a first separated image for the first molecule based on the first unseparated image, and generate a second separated image for the second molecule based on the first unseparated image and the second unseparated image.

EFFECT

According to some example embodiments, there may be provided an image processing method and system for removing autofluorescence and cross-reactivity of antibodies using a signal separation algorithm.

BRIEF DESCRIPTION OF DRAWINGS

FIGS. 1 to 4 are drawings for explaining the concept of **E**rasing bleed-through signals from antibody cross-**R**eactivity and **A**utofluore**S**cence **U**sing **R**Epeated Gram-Schmidt orthogonalization (ERASURE) according to an example embodiment of the present invention.

FIGS. 5 to 8 are drawings for validating separation of bleed-through signals in antibody cross-reactivity using ERASURE in an example embodiment of the present invention.

FIGS. 9 to 11 are drawings illustrating examples of multi-color imaging using antibodies derived from the same host species through ERASURE in an example embodiment of the present invention.

FIGS. 12 to 17 are drawings for explaining autofluorescence property and autofluorescence removal characteristics using ERASURE in an example embodiment of the present invention.

FIGS. 18 to 20 are drawings for explaining examples of simultaneously removing signals from autofluorescence, antibody cross-reactivity, and spectral overlap of fluorophores in an example embodiment of the present invention.

FIG. 21 is a block diagram illustrating an example of an internal configuration of an electronic device implemented as a computer device in an example embodiment of the present invention.

FIG. 22 is a flowchart illustrating an example of an image processing method according to an example embodiment of the present invention.

BEST MODE

The present invention may be modified in various manners and may have various example embodiments, so, hereinafter, specific example embodiments will be described in detail based on the accompanying drawings.

When it is determined that detailed description related to the related known art may obscure the gist of the present invention in describing the present invention, the detailed description is omitted.

Spatial proteomics investigates three-dimensional (3D) organization of cells and microenvironments of the cells in very heterogenous systems. Multi-fluorescence imaging has been widely used in this field, but the limited availability of antibody host species and high levels of autofluorescence have hindered the effective multi-imaging experimental design. Current solutions to address such issues often involve specialized chemicals or multiplexed imaging rounds, which limits broader applicability.

An example embodiment of the present invention presents Erasing bleed-through signals from antibody cross-Reactivity and AutofluoreScence Using REpeated Gram-Schmidt orthogonalization (ERASURE) (hereinafter, 'ERASURE'), a solution that overcomes the limited availability of antibody host species and alleviates the autofluorescence issue using an image processing technique. ERASURE may iteratively apply Gram-Schmidt (GS) orthogonalization to an image acquired in a single imaging round, so may remove bleed-through between a plurality of biometric molecule signals that occur in response to using a plurality of antibodies produced from the same host species, and may separate the same as each biometric molecule's own signal. Also, ERASURE may also remove bleed-through signals mixed with self-luminescence signals and fluorophores added to label specific biometric molecules inside the specimen from the outside in specimen with high self-luminescence, such as formalin-fixed paraffin-embedded (FFPE) samples, and may separate the same as a signal of each fluorophore. ERASURE enables the use of high autofluorescence samples by selecting antibodies regardless of host species and using the existing reagents, antibodies, and experimental procedures.

ERASURE may remove bleed-through signals caused by the use of primary antibodies derived from the same host species. Here, ERASURE may remove bleed-through signals from antibody cross-reactivity and autofluorescence using GS orthogonalization. ERASURE may acquire all images by performing a single imaging round and may effectively remove bleed-through signals arising from both antibody cross-reactivity and autofluorescence through repeated matrix decomposition. In the process of removing these undesired signals, GS orthogonalization, a well-established method extensively used in linear algebra for matrix decomposition, may be employed.

In the following, it is demonstrated that ERASURE allows six-color imaging of mouse brain using only rabbit-derived primary antibodies and may remove autofluorescence signals from FFPE samples. Also, it is demonstrated that removal of antibody cross-reactivity and autofluorescence may be simultaneously conducted using ERASURE. Also, it is demonstrated that ERASURE may be successfully used in conjunction with PICASSO, a technique for removing signals of spectrally overlapping fluorophores in a state in which reference emission spectra are absent in a mouse brain slice.

General working principle of ERASURE

In ERASURE, the same mathematical approach may be employed to remove all of bleed-through signals arising from antibody cross-reactivity and signals caused by autofluorescence. However, distinct staining and imaging protocols are followed to deal with these two sources of undesired signals. Initially, ERASURE may be used to remove bleed-through signals caused by antibody cross-reactivity

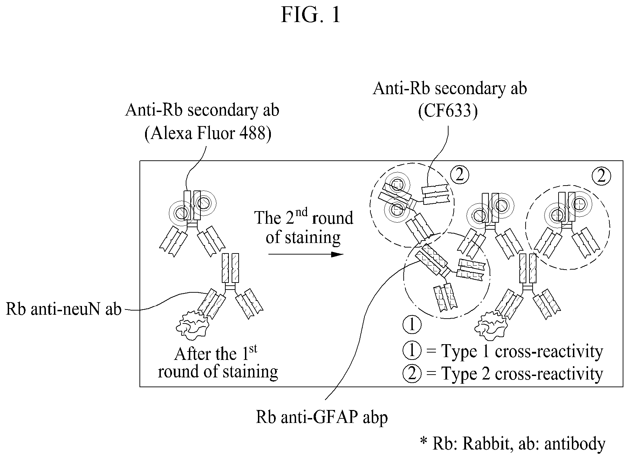

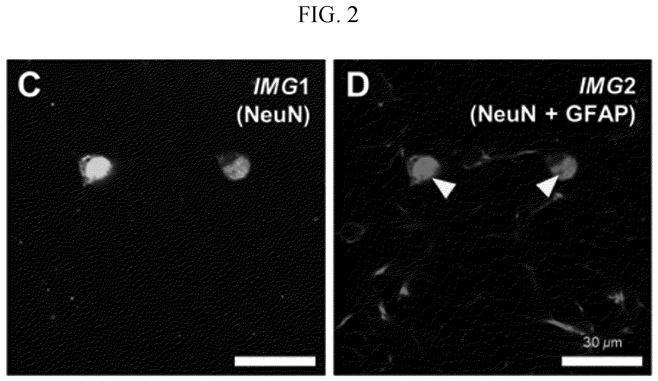

FIGS. 1 to 4 are drawings for explaining the concept of ERASURE according to an example embodiment of the present invention. Initially, FIG. 1 illustrates two types of antibody cross-reactivity. In ERASURE, proteins that are imaged may be stained in a serial manner. For example, after applying a primary antibody against a first target protein (e.g., rabbit anti-neuronal nucleus [NeuN]) to specimens, a secondary antibody that contains a fluorophore (e.g., Alexa Fluor 488) is applied (A of FIG. 1). Then, after applying a primary antibody against the second target protein (e.g., rabbit anti-glial fibrillary acidic protein (GFAP), a secondary antibody that contains a different fluorophore (e.g., CF633) is applied (B of FIG. 1). Then, two images are acquired from Alexa Fluor 488 and CF633 channels. In this process, two types of antibody cross-reactivity may occur. Type 1 cross-reactivity occurs when the primary antibody treated in the second round binds to the secondary antibody treated in the first round, whereas type 2 cross-reactivity occurs when the secondary antibody treated in the second round binds to the primary antibody treated in both the first and second rounds (② of B of FIG. 1). It was experimentally confirmed that antibody cross-reactivity caused significant bleed-through signals between two detection channels. FIG. 2 illustrates an example of antibody cross-reactivity. In C of FIG. 2, NeuN was visualized by Alexa Fluor 488 using a 488-nm laser, and in D of FIG. 2, GFAP was visualized by CF633 using a 637-nm laser. Due to antibody cross-reaction, NeuN signals were also observed (triangular arrows in D of FIG. 2). That is, only a NeuN (neuron marker) signal was detected in the first image (IMG1) acquired in the Alexa Fluor 488 channel (C of FIG. 2), and both NeuN and GFAP (astrocyte marker) signals were detected in the second image (IMG2) acquired in the CF633 channel (D of FIG. 2). It is hypothesized that, if NeuN signals in IMG2 and IMG1 have exactly the same brightness, an image of only GFAP may be acquired simply by subtracting IMG1 from IMG2. However, due to the use of different fluorophores, it is very difficult to acquire IMG2 in which the NeuN signal exactly matches one in IMG1. Over-subtraction may cause a reduction in fluorescence intensity in the GFAP channel in which the spatial expressions of NeuN and GFAP overlap. On the other hand, over-subtraction leaves residual signals in IMG2, which may be misinterpreted as a GFAP signals.

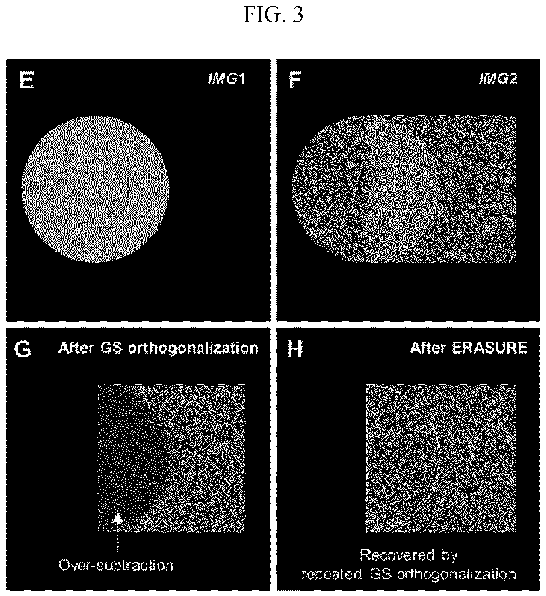

To solve this issue, a computational algorithm capable of accurately subtracting a NeuN signal from IMG2 is devised. In general, the ERASURE algorithm may subtract a protein A signal from a mixed image that includes both protein A and protein B signals. This may be achieved by iteratively applying GS orthogonalization, resulting in an image that contains only the protein B signal. GS orthogonalization produces an orthogonal set from an arbitrary vector set using projection of one vector onto another vector, regardless of the orthogonality of the original vector set. Therefore, if IMG1 that contains only the protein A signal is acquired (E of FIG. 3), the protein B signal may be extracted from IMG2 in which protein A and B signals coexist (F of FIG. 3). Mathematically, a vector orthogonal to IMG1 may be found as shown in Equation 1 below.

[Equation 1]

$$IMG2^{\perp IMG1} = IMG2 - \left(\frac{IMG2 \cdot IMG1}{|IMG1|^2}\right)IMG1$$

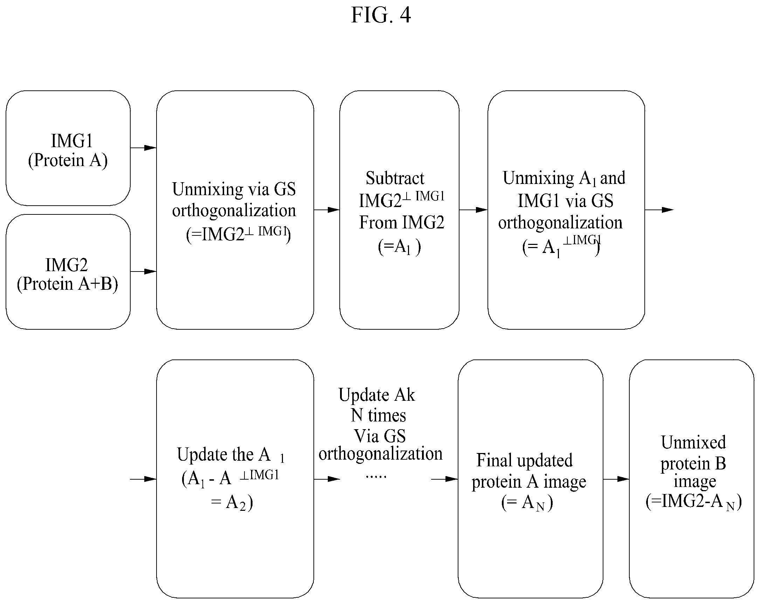

To acquire accurate results, relying solely on $IMG2^{\perp IMG1}$ is not possible, which may be acquired through GS orthogonalization of IMG1 and IMG2. This is because the region in which proteins A and B coexist is over-subtracted, resulting in incorrect results (G of FIG. 3). ERASURE refers to a technique for producing precise results by gradually recovering the over-subtracted region through repeated GS orthogonalization (H of FIG. 3). FIG. 3 is an example of separating a bleed-through signal using GS orthogonalization and ERASURE. E of FIG. 3 shows IMG1 that contains only a circle signal, and F of FIG. 3 shows IMG2 that contains both circle and rectangle signals. Also, an over-subtracted region (dotted arrow) after GS orthogonalization is observed in G of FIG. 3, and H of FIG. 3 shows the over-subtracted region being recovered (dotted semicircle) after applying ERASURE. FIG. 4 illustrates an example of a process of ERASURE. After performing GS orthogonalization between IMG1 and IMG2 vectors, ERASURE starts by subtracting $IMG2^{\perp IMG1}$ from IMG2 as shown in Equation 2 below.

[Equation 2]

$$IMG2 - IMG2^{\perp IMG1} = A_1$$

Then, $A_1$ with the same pattern as IMG1 may be acquired, but the signal intensity is higher than in IMG1 in regions in which protein A and protein B overlap due to over-subtraction in $IMG2^{\perp IMG1}$. To solve this, ERASURE conducts GS orthogonalization again with IMG1 for $A_1$ as shown in Equation 3 below.

[Equation 3]

$$A_1^{\perp IMG1} = A_1 - \left(\frac{A_1 \cdot IMG1}{|IMG1|^2}\right) IMG1$$

Also, updated $A_2$ with slightly reduced intensity in the overlapping region may be acquired by subtracting $A_1^{\perp IMG1}$ from $A_1$ as shown in Equation 4 below.

[Equation 4]

$$A_2 = A_1 - A_1^{\perp IMG1}$$
$$= A_1 - \left(A_1 - \left(\frac{A_1 \cdot IMG1}{|IMG1|^2}\right) IMG1\right)$$
$$= \left(\frac{A_1 \cdot IMG1}{|IMG1|^2}\right) IMG1$$

Equation 3 and Equation 4 are iteratively applied N times until unit vectors of IMG1 and $A_N$ become parallel. Through this process, over-subtracted regions may be gradually restored. A final image ($A_N$) of protein A may be expressed as Equation 5 below.

[Equation 5]

$$A_N = \left(\frac{A_{N-1} \cdot IMG1}{|IMG1|^2}\right) IMG1,$$

$$\left(\lim_{k \to N} \frac{IMG1}{|IMG1|} \cdot \frac{A_k}{|A_k|} \approx 1, \quad N = \text{iteration number}\right)$$

Therefore, ERASURE allows acquisition of $A_N$, from which both the signal intensity and the pattern of protein A in IMG2 may be accurately determined. Finally, by recovering the over-subtracted regions after $A_N$ is subtracted from IMG2, ERASURE provides a more price image of protein B by recovering the over-subtracted regions than when performing GS orthogonalization only once.

Validation of ERASURE

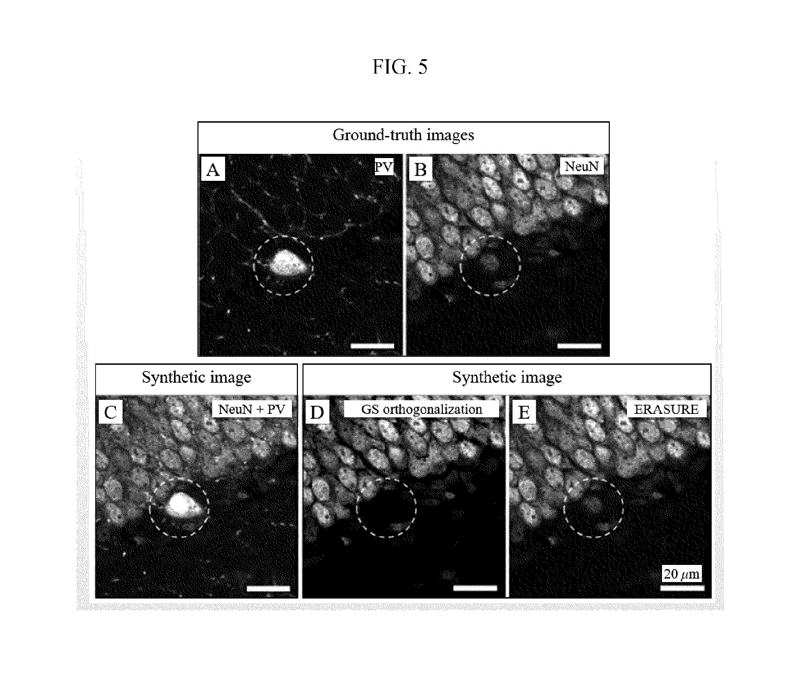

To evaluate the performance of the ERASURE algorithm in removing bleed-through signals caused by antibody cross-reactivity, simulations were conducted by acquiring133 two-channel images in which each channel contained a single protein signal and then, by generating synthetic mixed images that contain signals of two proteins. Then, the mixed images were fed to the ERASURE algorithm and the Pearson Correlation Coefficients (PCC) were calculated by comparing resulting images to ground-truth images. The resulting images showed high correlation with the ground-truth images with the average PCC of 0.989. Then, the performance of the ERASURE algorithm was tested in the case of two spatially overlapping proteins. FIGS. 5 to 8 are drawings for validating separation of bleed-through signals in antibody cross-reactivity using ERASURE in an example embodiment of the present invention. FIG. 5 shows simulation results of separating spatially overlapping proteins, A of FIG. 5 is a ground-truth image, showing only a PV signal, B of FIG. 5 is a ground-truth image, showing only a NeuN signal, and C of FIG. 5 is a mixed image acquired by synthesizing A of FIG. 5 and B of FIG. 5 at an intensity ratio of 1:1. Also, D of FIG. 5 is a resulting image acquired after applying A of FIG. 5 and C of FIG. 5 to GS orthogonalization, and shows that the NeuN signal was over-subtracted in a PV and NeuN overlapping region (dotted circled region). Also, E of FIG. 5 is an image after applying ERASURE and, here, the NeuN signal is visible in the overlapping region (dotted circled region). In more detail, whether the ERASURE algorithm may reliably separate PV and NeuN signals in overlapping regions (dotted circled regions in A and B of FIG. 5) is determined by selecting parvalbumin (PV; interneuron marker) and NeuN antibodies, both of which bind to interferon in the mouse brain, and by acquiring ground-truth images. After generating nine synthetic images each containing both PV and NeuN signals with different intensity ratios, as shown in C of FIG. 5, how the performance of one-time GS orthogonalization and the ERASURE algorithm differed was examined. As a result of comparing the resulting image and the ground-truth images, the NeuN signals were over-subtracted in the overlapping region after GS orthogonalization (D of FIG. 5), whereas the ERASURE algorithm preserved the NeuN signals in the corresponding region (E of FIG. 5). Also, all correlations between mixed and ground-truth images generated by the ERASURE algorithm had PCC values greater than 0.999.

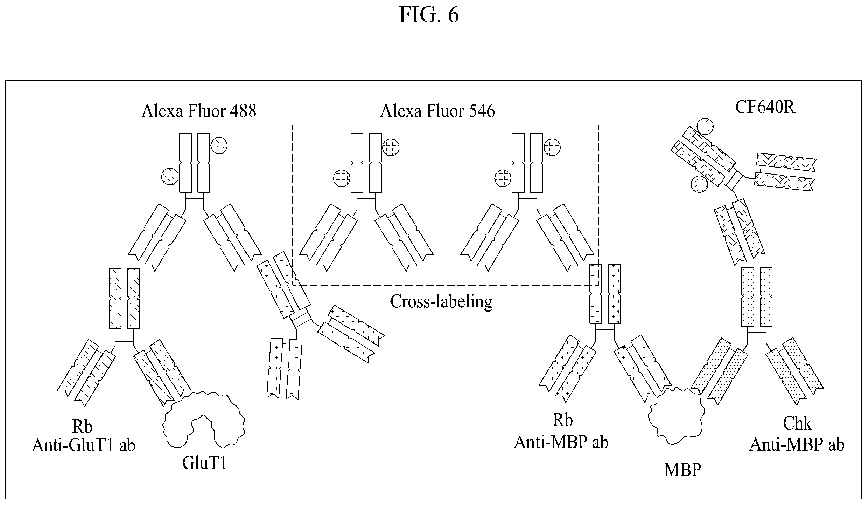

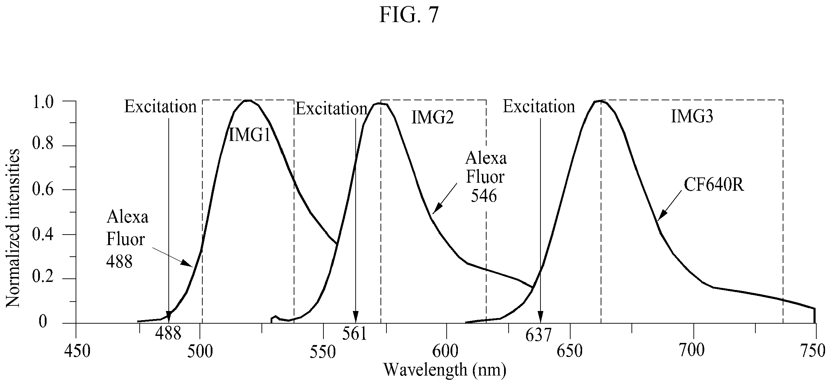

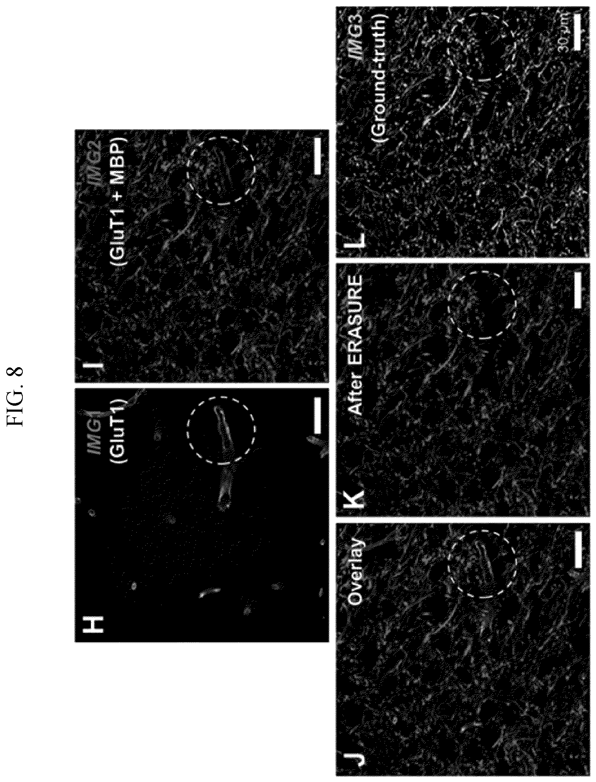

Subsequently, the performance of the ERASURE algorithm was experimentally validated in a mouse brain slice. In the first round of staining, the slice was sequentially incubated with a rabbit anti-glucose transporter 1 (GluT1; blood vessel marker) antibody and a secondary antibody containing Alexa Fluor 488 (FIG. 6). FIG. 6 is a schematic diagram of experiments in which rabbit primary antibodies against GluT1 and MBP were cross-labeled with an Alexa Fluor 546-conjugated secondary antibody (dashed box in FIG. 6). FIG. 6 shows emission spectra of three fluorophores (Alexa Fluor 488, Alexa Fluor 546, and CF640R) and detection ranges used in this experiment, and Alexa Fluor 488 was excited by a 488-nm laser and imaged in the first detection range (dashed box of IMG1). Alexa Fluor 546 was excited by a 561-nm laser and imaged in the second detection range (dashed box of IMG2). CF640R was excited with a 637-nm laser and imaged in the third detection range (dashed box of IMG3). Then, the sample was treated with a rabbit anti-myelin basic protein (MBP; myelin marker) antibody and an anti-rabbit secondary antibody containing Alexa Fluor 546. To acquire a ground-truth image of MBP, a chicken anti-MBP antibody and an anti-chicken secondary antibody containing CF640R were used. Anti-GluT1 and MBP primary antibodies originating from rabbits bound to two primary antibodies (dashed box of FIG. 6) with the anti-rabbit secondary antibody containing Alexa Fluor 546. The sample was imaged using 488-nm, 561-nm, and 637-nm excitation lasers, and three images (IMG1, IMG2, and IMG3) were acquired in three detection channels (dashed boxes in FIG. 7). H of FIG. 8 is IMG1, showing only GluT1, I of FIG. 8 is IMG2, showing both GluT1 and MBP. J of FIG. 8 is an image acquired by overlaying H and I, and K of FIG. 8 shows that the GluT1 signal was removed after applying ERASURE (dotted circled region). L of FIG. 8 is IMG3 (ground-truth image) and shows an example showing only MBP. An image acquired by overlaying IMG1 (H of FIG. 8) and IMG2 (I of FIG. 8) showed that antibody cross-labeling caused signal cross talk in the second detection channel (dashed circles in I and J of FIG. 8). As such, it was confirmed that the ERASURE algorithm may subtract the GluT1 signal from IMG2, and the resulting image (K of FIG. 8) highly correlated with the ground-truth image IMG3 (L of FIG. 8).

Hereinafter, results regarding whether a serial staining process caused a reduction in antibody signal intensity, potentially attributable to antibody cross-reactivity, are described. A portion of primary antibodies that target second, third, and other target proteins is captured by the secondary antibody present in a specimen. This occurs due to type 1 antibody cross-reactivity (D of FIG. 2), leading to decreasing antibody signals. To study this effect, a mouse brain slice was divided into right and left hemispheres. One of them was labelled with rabbit anti-GFAP primary antibody and Alexa Fluor 546-conjugated anti-rabbit secondary antibody, and the other one was sequentially labeled with rabbit anti-NeuN primary antibody, Alexa Fluor 488-conjugated anti-rabbit secondary antibody, rabbit anti-GFAP primary antibody, and Alexa Fluor 546-conjugated anti-rabbit secondary antibody. Subsequently, a hippocampal region was imaged using the 561-nm laser, and the signal intensity of 100 astrocytes was measured. The results showed that antibody cross-labeling did not cause a notable decrease in the signal intensity of the target protein.

Multi-color imaging using antibodies originating from the same host species

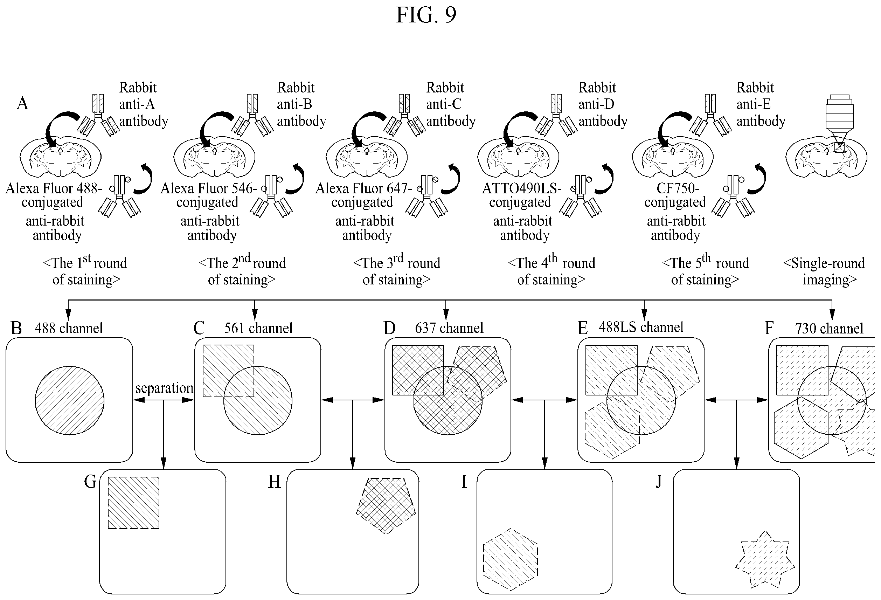

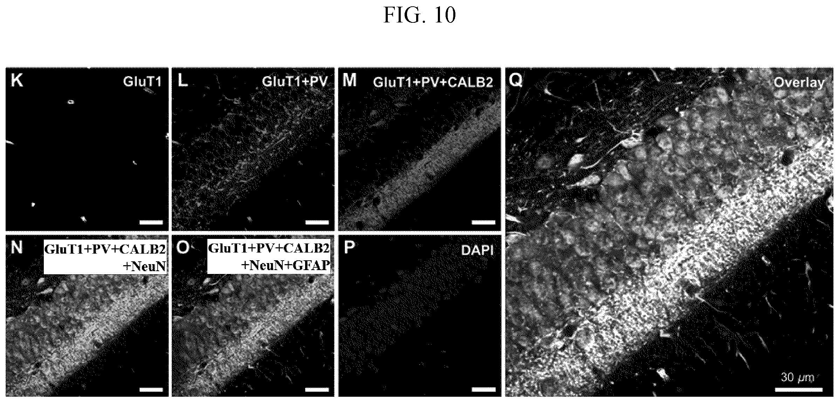

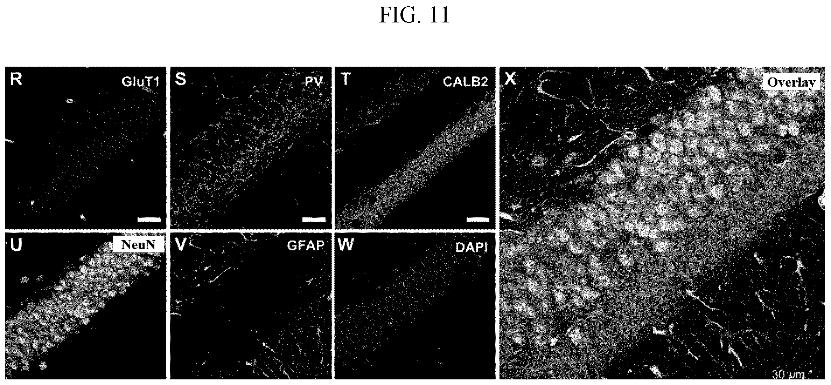

Then, the multi-color imaging capability of ERASURE was validated. FIGS. 9 to 11 are drawings illustrating examples of multi-color imaging using antibodies originating from the same host species through ERASURE in an example embodiment of the present invention. A to J of FIG. 9 are schematic diagrams of multi-color imaging using ERASURE. A of FIG. 9 represents an example in which proteins A, B, C, D, and E were stained using rabbit-derived primary antibodies, and each was labeled with secondary antibodies containing five different fluorophores (Alexa Fluor 488, Alexa Fluor 546, Alexa Fluor 647, ATTO490LS, and CF750). In a single imaging round, five mixed images were acquired. In more detail, an experiment of sequentially labeling proteins A, B, C, D, and E with primary antibodies originating from the same host species and labeling each of the same with secondary antibodies containing five different fluorophores was organized (A of FIG. 9). Subsequently, the sample was imaged in a single round (B to F of FIG. 9). B of FIG. 9 is a mixed image showing protein A, C of FIG. 9 is a mixed image showing proteins A and B, D of FIG. 9 is a mixed image showing proteins A, B, and C, E of FIG. 9 is a mixed image showing proteins A, B, C and D, and F of FIG. 9 is a mixed image showing proteins A, B, C, D, and E. Although a protein A signal was distinguishable in the first detection channel (B of FIG. 9), bleed-through signals appeared in other channels due to the use of the same host antibodies in previous staining rounds (C to F of FIG. 9). Individual protein images were acquired by performing ERASURE between images acquired for each channel (G to J of FIG. 9). G of FIG. 9 represents protein B, H of FIG. 9 represents protein C, I of FIG. 9 represents protein D, and J of FIG. 9 represents protein E. To achieve this, a mouse brain slice was subjected to serial staining using antibodies, such as rabbit primary antibody against GluT1, Alexa Fluor 488-conjugated secondary antibody against rabbit, rabbit primary antibody for PV, CF568-conjugated secondary antibody against rabbit, rabbit primary antibody against calb2, Alexa Fluor 647-conjugated secondary antibody, rabbit primary antibody against NeuN, ATTO490LS-conjugated secondary antibody, rabbit primary antibody against GFAP, and CF750-conjugated secondary antibody against rabbit. Also, the brain slice was stained with 4',6-diamidino-2-phenylindole (DAPI). After sixth staining and single imaging rounds, multichannel mixed images were acquired and fed to the ERASURE algorithm (K to Q of FIG. 10). K to Q of FIG. 10 are mixed images acquired after sixth staining, and K of FIG. 9 shows GluT1, L of FIG. 9 shows GluT1 and PV, M of FIG. 9 shows GluT1, PV, and CALB2, N of FIG. 9 shows GluT1, PV, CALB2, and NeuN, O of FIG. 9 shows GluT1, PV, CALB2, NeuN, and GFAP, and P of FIG. 9 shows DAPI. Also, Q of FIG. 9 is an image acquired by overlaying K on P. The mixed images were successfully separated into images of the respective proteins after ERASURE (R to X of FIG. 11). R to X of FIG. 11 are separated images after ERASURE, R of FIG. 11 shows GluT1, S of FIG. 11 shows PV, T of FIG. 11 shows CALB2, U of FIG. 11 shows NeuN, V of FIG. 11 shows GFAP, W of FIG. 11 shows DAPI, and X of FIG. 11 is an image acquired by overlaying R on W. ERASURE is not restricted to antibody staining. It is confirmed that ERASURE may separate signal bleed-through caused by streptavidin-biotin cross-reactivity.

Autofluorescence removal using ERASURE

The performance of the ERASURE algorithm in removing bleed-through signals caused by autofluorescence is described. Autofluorescence originates from diverse fluorescent sources, such as the extracellular matrix, blood cells, and lipofuscin, and each source has a unique emission spectrum due to its intrinsic properties. Initially, the autofluorescence features of various organs were investigated by measuring their autofluorescence emission spectra. Various different organs were examined, including the adrenal gland, brain (gray and white matter), breast, cerebellum, colon, endometrium (proliferative), endometrium (secretory), esophagus, heart, kidney (cortex), kidney (medulla), liver, lung, lymph node, ovary, pancreas, placenta, prostate, rectum, salivary gland, skeletal muscle, small intestine, spleen, stomach, testis, thymus, thyroid, tonsil, and urinary bladder. Each organ had a unique autofluorescence emission spectrum, which may be attributed to the varying compositions and expression levels of intrinsic autofluorescence components. Then, whether commercially available autofluorescence quenchers may effectively eliminate all kinds of autofluorescence signals and be compatible with antibody labeling was tested. In some cases, the quenchers failed to completely remove the signals from all intrinsic autofluorescence sources. Also, it was found that using detergents, such as Triton-X or saponin, may hinder binding of quenchers to auto-fluorescent biomolecules or detach them from biomolecules. Also, it was confirmed that quenchers may lead to decreasing a signal-to-noise ratio by binding specific fluorophores and decreasing their signal intensity.

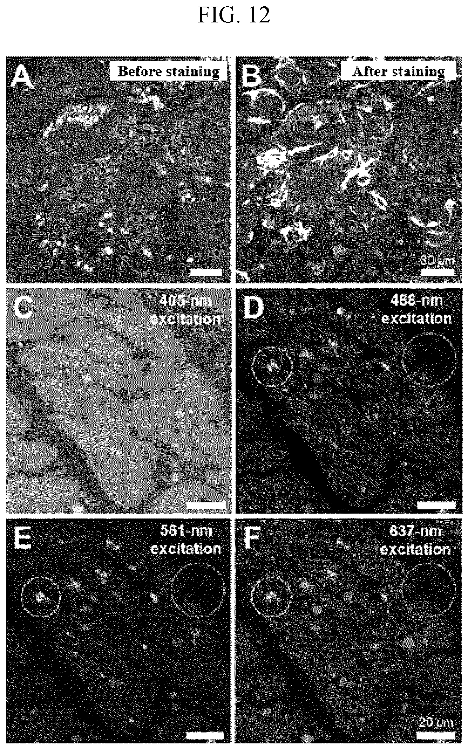

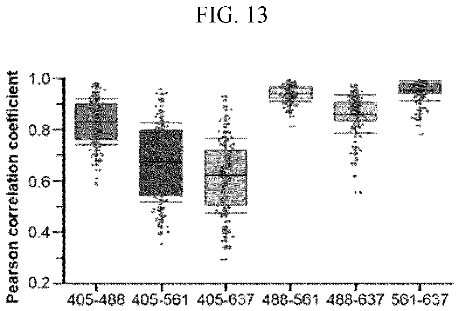

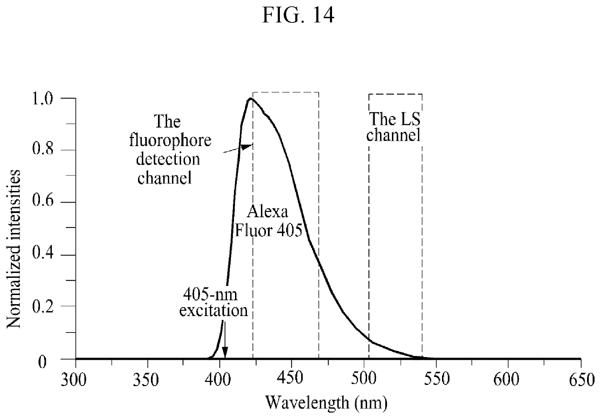

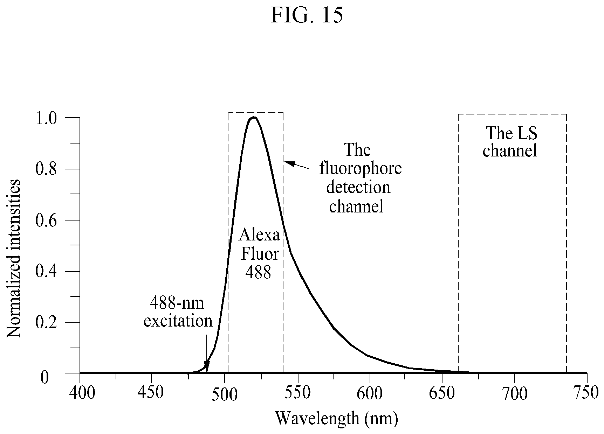

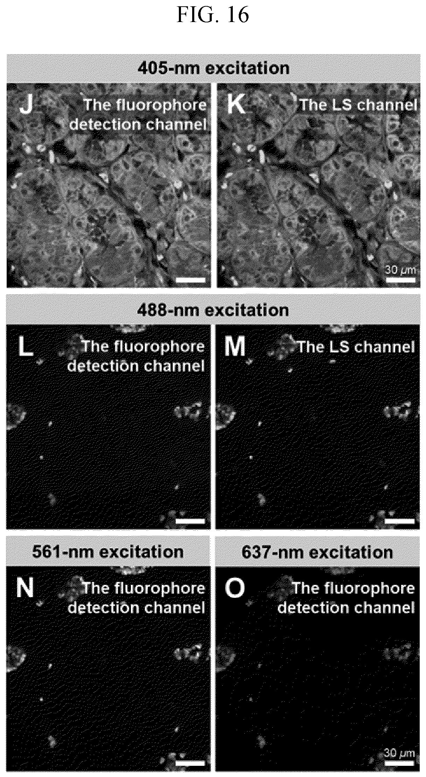

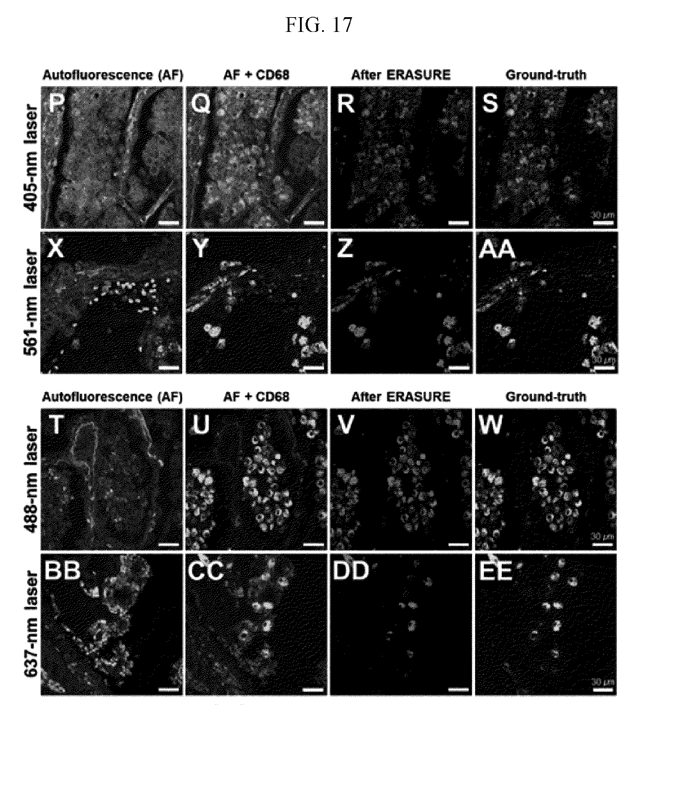

Given that the ERASURE algorithm is based on subtracting bleed-through signals from a mixed image, autofluorescence signals highly expressed in FFPE tissues may also be separated. FIGS. 12 to 17 are drawings for explaining autofluorescence property and autofluorescence removal characteristics using ERASURE in an example embodiment of the present invention. A and B of FIG. 12 are images of autofluorescence signal before and after antibody staining. A human FFPE stomach sample was labeled with a rabbit anti-$\alpha$ smooth muscle actin primary antibody and an anti-rabbit Alexa Fluor 546 secondary antibody. A of FIG. 12 is an autofluorescence image acquired using a 488-nm laser before staining. B of FIG. 12 is a mixed image acquired using a 561-nm laser after staining. It can be seen that the autofluorescence signal is still present (triangular arrows). C to F of FIG. 12 are autofluorescence images acquired using four excitation lasers for a human FFPE heart sample. Excitation wavelengths are 405 nm in C of FIG. 12, 488 nm in D of FIG. 12, 561 nm in E of FIG. 12, and 637 nm in F of FIG. 12. FIG. 13 shows PCC between autofluorescence images acquired using different excitation wavelengths. In the graph of FIG. 13, the wavelength of a first box ranges from 405 nm to 488 nm (average = 0.831, S.D. = 0.090), the wavelength of a second box ranges from 405 nm to 561 nm (average = 0.673, S.D. = 0.155), the wavelength of a third box ranges from 405 nm to 637 nm (average = 0.621, S.D. = 0.145), the wavelength of a fourth box ranges from 488 nm to 561 nm (average= 0.939, S.D.= 0.030), the wavelength of a fifth box ranges from 488 nm to 637 nm (average = 0.860, S.D. = 0.074), and the wavelength of a sixth box ranges from 561 nm to 637 nm (average = 0.952, S.D. = 0.039). Upper and lower bounds of each box represent 25th and 75th percentiles, respectively, while middle lines represent mean values. The whiskers represent the standard deviation. FIGS. 14 and 15 show approaches to acquire images that include only an autofluorescence signal after staining. FIG. 14 shows the normalized emission spectrum of Alexa Fluor 405 and the fluorophore detection channel and the LS channel, and FIG. 15 shows the normalized emission spectrum of Alexa Fluor 488 and the fluorophore detection channel and the LS channel. J and K of FIG. 16 are autofluorescence images of a human FFPE stomach sample acquired using a 405-nm laser in two detection channels shown in H. J of FIG. 16 is an image acquired in the fluorophore detection channel, and K of FIG. 16 is an image acquired in the LS channel. L and M of FIG. 16 are autofluorescence images of a human FFPE tonsil sample using a 488-nm laser in two detection channels shown in I. L of FIG. 16 is an image acquired in the fluorophore detection channel, and M of FIG. 16 is an image acquired in the LS channel. The same sample shown in N and O of FIG. 16 was imaged using two excitation lasers in the fluorescence detection channel. N of FIG. 16 shows an image acquired using a 561-nm laser, and O of FIG. 16 shows an image acquired using a 637-nm laser. P to EE of FIG. 17 are examples of experimental validation of autofluorescence removal using ERASURE. Human FFPE lung cancer (adenocarcinoma) samples were labeled with a primary antibody against CD68. In P to S of FIG. 17, CD68 was visualized using Alexa Fluor 405 and imaged with a 405-nm laser. P of FIG. 17 is an autofluorescence image acquired in the LS channel. Q of FIG. 17 is a mixed image acquired in the fluorophore detection channel and shows both autofluorescence and CD68 signals. R of FIG. 17 is an image after applying ERASURE, and S of FIG. 17 shows a ground-truth image. T to W of FIG. 17 are similar to P to S of FIG. 17, but show results using Alexa Fluor 488 and a 488-nm laser. X to AA of FIG. 17 are similar to P to S of FIG. 17, but show results using Alexa Fluor 546 and a 561-nm laser. BB to EE of FIG. 17 are similar to P to S of FIG. 17, but show results using Alexa Fluor 647 and a 637-nm laser.

Initially, how autofluorescence affected fluorescence imaging in human FFPE tissues before and after antibody staining was to be examined (A and B of FIG. 12). The autofluorescence signal persisted even after a sample was treated with primary and secondary antibodies (triangular arrows in A and B of FIG. 12). Then, it was confirmed that ERASURE may be used to eliminate autofluorescence signals from a human FFPE sample. An autofluorescence image of a sample was acquired prior to staining with antibodies. Subsequently, the sample was stained with a primary antibody against α-smooth muscle actin and an Alexa Fluor 488-conjugated secondary antibody, and acquired an image containing both autofluorescence and α-smooth muscle actin signals. The two images were used to see if the ERASURE algorithm may eliminate the autofluorescence signal from the latter image. The algorithm effectively removed the autofluorescence from the second image, showing only α-smooth muscle actin. However, this approach requires that specimens be imaged twice-before and after antibody staining-adding complexity to the overall imaging procedure. In addition, it was observed that changes in the field of view in x, y, and z axes during sample positioning on a microscope stage may result in pixel and protein structure shifts in images acquired before and after staining. While such shifts may be compensated for by a robust registration algorithm using a fiducial marker, accurate sample positioning and finding the exact same region are very difficult and sometimes impractical without a microscope that has stable performance in adjusting stage alignment.

To address this issue, whether the ERASURE algorithm may successfully remove autofluorescence in a single imaging process was examined. To achieve this, images of antibody-stained specimens were acquired in two detection channels. One channel encompassed the emission peak of the fluorophore used, while the other had a wavelength of 80 nm or more, which excluded any signals from the fluorophore. For simplicity, hereinafter, the first and second channels are referred to as "fluorophore detection channel" and "long-shift (LS) channel," respectively. The image acquired in the fluorophore detection channel included both antibody signals and autofluorescence, whereas the image acquired in the LS channel included only autofluorescence. Although these images were acquired using two different detection channels with the wavelength of 80 nm or more, it was hypothesized that autofluorescence patterns in the fluorophore detection and LS channels would be identical. To test this hypothesis, our initial investigation focused on examining how the autofluorescence pattern of FFPE heart slices changed across wavelengths. This was accomplished by imaging unstained FFPE heart slices using one of four standard excitation lasers (405 nm, 488 nm, 561 nm, and 637 nm) in the fluorophore detection channels of the corresponding excitation lasers (C to F of FIG. 12). Results of analyzing the autofluorescence patterns showed that the image acquired with the 405-nm excitation laser (C of FIG. 12) exhibited notably different patterns from the other images (D to F of FIG. 12). C of FIG. 12 did not show a signal of blood cells (small dotted circled region); however, an extracellular matrix signal (large dotted circled region) was considerably brighter than in the other images (D to F of FIG. 12). Subsequently, a pair were selected from among four images for all conceivable pairs and the PCC values were calculated (FIG. 13). Images acquired with 405-nm excitation had comparatively low PCC values: 0.831, 0.673, and 0.621 for each pair, whereas images acquired with 488-nm excitation had comparatively high PCC values: 0.939, 0.860, and 0.952 for each pair (157 measurements from eight samples of six organ types). These results were consistent with visual observations (C to F of FIG. 12). Also, it was confirmed that only two images, one acquired using the 405-nm excitation laser and the other acquired using the 488-nm laser, were sufficient to remove autofluorescence signals from the four images. Specifically, the autofluorescence in the images acquired with 488-nm, 561-nm, and 637-nm excitations were capable of being effectively eliminated by employing a single autofluorescence image of the FFPE heart sample acquired using the 488-nm excitation laser.

Then, changes in autofluorescence patterns according to the detection ranges were investigated. As shown in FIGS. 13 and 15, images of unstained FFPE slices were acquired using either the 405-nm or 488-nm excitation laser. For each excitation laser, images were acquired in the fluorophore detection and LS channels (J to M of FIG. 16). Also, images were acquired using 561-nm and 637-nm excitation lasers in the fluorophore detection channels (i.e., 561-nm and 637-nm channels) and compared with the autofluorescence patterns acquired using the 488-nm excitation laser (N and O of FIG. 16). Then, the correlations between the autofluorescence images acquired in the two channels were calculated for each excitation laser. The average correlation between two images acquired using the 405-nm laser in the two detection channels (FIG. 14) had a PCC value of 0.937 (1,011 measurements from 29 organs). The average correlation between two images acquired using the 488-nm laser in the two detection channels had a PCC value of 0.915 (1,066 measurements from 29 organs). Also, the autofluorescence images acquired in the LS channel of the 488-nm excitation laser showed high correlations with images acquired in the fluorophore detection channels of Alexa Fluor 546 and Alexa Fluor 647, with PCC values of 0.938 and 0.871 (1,006 measurements from 29 organs), respectively. These results supported two hypotheses. First, the autofluorescence present in the fluorophore detection channel of the 405-nm excitation laser was capable of being effectively eliminated using the image acquired in the LS channel of the 405-nm excitation laser. Second, the autofluorescence observed in the fluorophore detection channels of 488-nm, 561-nm, and 647-nm excitation lasers was capable of being removed by employing the image obtained in the LS channel.

Subsequently, the capability of ERASURE to remove autofluorescence across all visible light ranges in lung cancer (adenocarcinoma) was demonstrated. Four FFPE samples were stained with a CD68 antibody and then, labeled with secondary antibodies containing Alexa Fluor 405, Alexa Fluor 488, Alexa Fluor 546, and CF633, respectively. Since a 730-nm excitation laser rarely induced autofluorescence, CF750 was used to acquire ground-truth images. To acquire an autofluorescence image from a sample labeled with a secondary antibody containing Alexa Fluor 405, the 405-nm excitation laser and the LS channel of 405-nm laser were used (P of FIG. 17). Also, three autofluorescence images were acquired from three samples, each labeled with secondary antibodies containing Alexa Fluor 488, Alexa Fluor 546, and CF633, using the 488-nm excitation laser in the LS channel of 488-nm laser (T, X, and BB of FIG. 17). The samples were imaged using four excitation lasers (405 nm, 488 nm, 561 nm, and 637 nm) in the fluorophore detection channels, and mixed images that contained both autofluorescence and CD68 signals were acquired (Q, U, Y, and CC of FIG. 17). Then, autofluorescence images and mixed images were applied to the ERASURE algorithm. The ERASURE-processed images (R, V, Z, and DD of FIG. 17) matched the ground-truth images well (S, W, AA, and EE of FIG. 17).

Simultaneous removal of signal cross talk from antibody cross-labeling and autofluorescence

Finally, an attempt was made to simultaneously remove signal bleed-through from antibody cross-reactivity and autofluorescence using ERASURE.

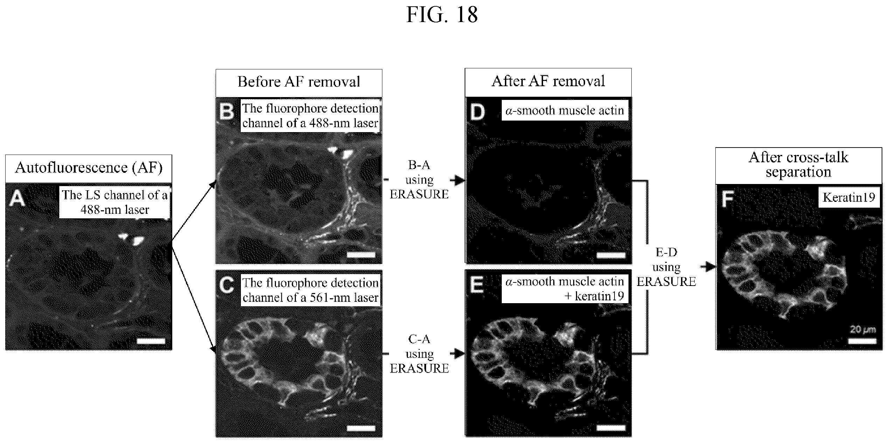

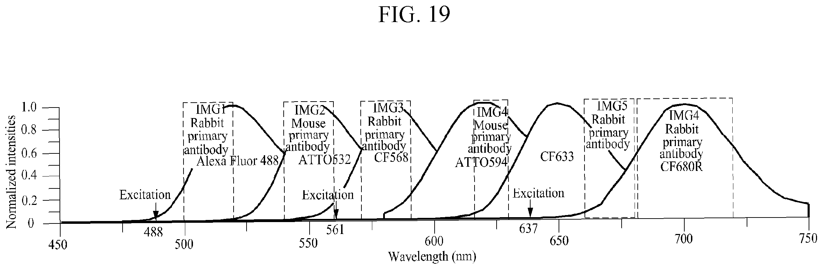

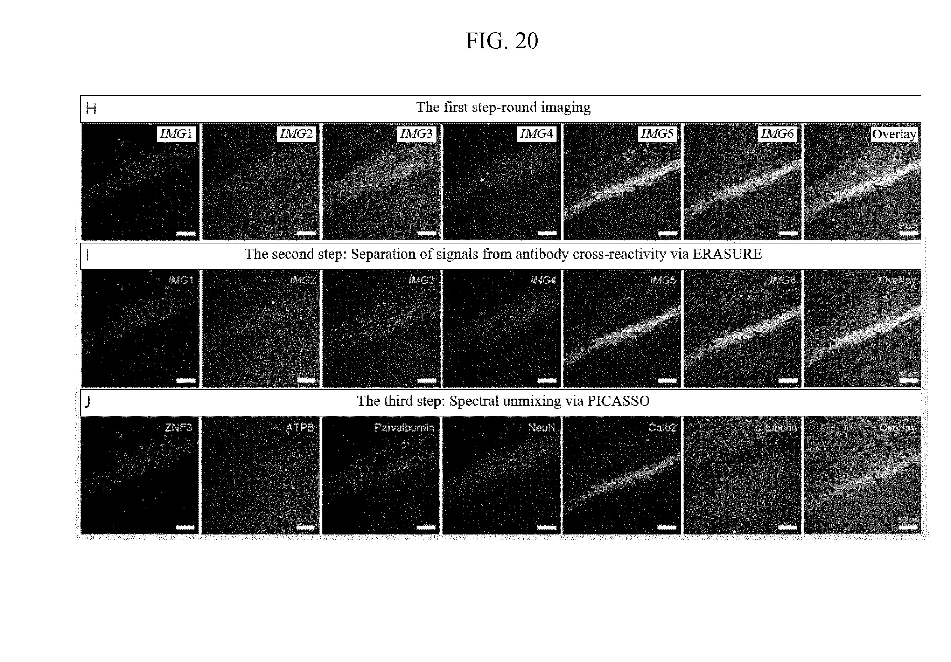

FIGS. 18 to 20 are drawings for explaining examples of simultaneously removing signals from autofluorescence, antibody cross-reactivity, and spectral overlap of fluorophores in an example embodiment of the present invention. A to F of FIG. 18 are images of signals of autofluorescence and antibody cross-reactivity removal in a human FFPE ovary sample using ERASURE. A of FIG. 18 is an autofluorescence image acquired in the LS channel, and B of FIG. 18 is a mixed channel acquired in the fluorescence detection channel (488 nm channel). Autofluorescence and $\alpha$-smooth muscle actin signals are detected. C of FIG. 18 is mixed signal acquired in the fluorescence detection channel (561 nm channel), and autofluorescence, $\alpha$-smooth muscle actin, and keratin 19 signals are detected. D of FIG. 18 is an $\alpha$- smooth muscle actin image after removing the autofluorescence signal in B using ERASURE, and E of FIG. 18 is a mixed image that contains $\alpha$-smooth muscle actin and keratin 19 signals after removing the autofluorescence signal in C. F of FIG. 18 is keratin 19 image after removing signals from antibody cross-reactivity in E. FIGS. 19 and 20 show examples of simultaneously removing signals from antibody cross-reactivity and spectral overlap of fluorophores using ERASURE and PICASSO. FIG. 19 shows fluorophores' emission spectra, excitation lasers (black arrows), and detection channels (dotted boxes) used in this experiment. H of FIG. 20 shows a mixed image acquired after staining with the same host antibodies containing spectrally overlapping fluorophores. I of FIG. 20 is a mixed image after applying ERASURE. Bleed-through signals from antibody cross-reactivity are eliminated while signals from spectrally overlapping fluorophores are still present. J of FIG. 20 shows individual protein images after PICASSO, and mixed images shown in I of FIG. 20 were fed to the PICASSO algorithm. In more detail, for example, the human FFPE ovary sample was labeled with an anti-$\alpha$-smooth muscle actin antibody and a secondary antibody containing Alexa Fluor 488, followed by an anti-keratin antibody and a secondary antibody containing Alexa Fluor 546. Through this, three mixed images were acquired. One is an image with an autofluorescence signal only (A of FIG. 18), one is an image with both autofluorescence and $\alpha$-smooth muscle actin signals (B of FIG. 18), and one is an image with autofluorescence, $\alpha$-smooth muscle actin, and keratin19 signals (C of FIG. 18). Initially, the autofluorescence was removed from the mixed images. In particular, unlike the mixed images (B and C of FIG. 18), no autofluorescence was observed in the ERASURE-processed images (D and E of FIG. 18). After removing the autofluorescence, the images were applied again to the ERASURE algorithm to acquire an image containing only keratin19 from the image containing both $\alpha$-smooth muscle actin and keratin19 signals (F of FIG. 18). It was confirmed that ERASURE was capable of successfully eliminating the bleed-through signals arising from antibody cross-reactivity.

To enhance the multiplexed imaging capability of ERASURE, it was combined with PICASSO, a technique that may separate signals from spectrally overlapping fluorophores without using reference emission spectra. A mouse brain slice was stained with three rabbit-derived primary antibodies (i.e., against zinc finger 3 [ZNF3], PV, and calb2 proteins) and three mouse-derived primary antibodies (i.e., against adenosine triphosphate synthase subunit beta [ATPB], NeuN, and $\alpha$-tubulin proteins), and each protein was labeled with three pairs of two spectrally overlapping fluorophores, excited by 488-nm, 561-nm, and 637-nm lasers, respectively (H of FIG. 20). Then, the stained sample was imaged using a confocal microscope equipped with a spectral detector to adjust six detection ranges and to separate the signals of spectrally overlapping fluorophores using PICASSO (FIG. 19). Six images were acquired with highly mixed and overlapping protein signals, resulting from signal cross talk among IMG1, IMG3, and IMG5 due to rabbit-derived primary antibody cross-labeling and signal cross talk among IMG2, IMG4, and IMG6 due to mouse-derived primary antibodies cross-labeling (H of FIG. 20). Signal cross talk between IMG1 and IMG2, IMG3 and IMG4, and IMG5 and IMG6 due to the spectral overlap of fluorophores was also observable (H of FIG. 20). Six mixed images were fed to the ERASURE algorithm, which effectively removed the bleed-through signals from antibody cross-labeling (I of FIG. 20). Finally, the ERASURE-processed images were fed to the PICASSO algorithm. The resulting images contained only individual protein signals, respectively (J of FIG. 20). Overlaid images showed that signal cross talk due to cross-labeling and spectrally overlapping fluorophores was gradually removed using ERASURE and PICASSO (H to J of FIG. 20).

Meanwhile, removal of autofluorescence and removal of signal overlap due to antibody cross-reactivity are not limited to the aforementioned ERASURE algorithm and may be implemented as other similar algorithms as follows.

1. Mutual information minimization-based signal separation technique (Patent Application Nos: KR 10-2020-0088091, KR 10-2021-0089419, KR 10-2021-0090057, KR 10-2021-0171431, US17/132628, US17/445629, US17/567300, PCT/KR2021/011097, PCT/KR2021/018833, EP22150031.7, EP21192162.2)

The mutual information minimization-based signal separation technique refers to a signal separation technique based on the assumption that images with overlapping signals have a high amount of mutually shared information, and may separate each image into a unique signal of each fluorescent molecule or protein by minimizing mutual information of the images with overlapping signals.

2. Joint histogram-based signal separation technique (Patent Application Nos.: KR 10-2022-0096449, US17/931711)

A joint histogram refers to a signal intensity relationship of corresponding voxels on a plurality of images. Representative indices derived from the joint histogram include mutual information, Kullback-Leibler divergence, cross-entropy, and Rand-index. If at least one of the joint histogram indices is minimized, each of images containing mixed fluorescent molecule or protein signals may be separated in its unique signal.

3. Self-supervised neural unmixing-based signal separation technique (Patent Application Nos.: KR 10-2022-0109288, KR 10-2022-0150599, KR 10-2023-0052215, KR 10-2023-0113481, PCT/KR2023/012865)

The self-supervised neural unmixing-based signal separation technique refers to a self-supervised neural unmixing-based method that may iteratively evaluate dependency between two images with mixed signals and may perform unmixing by finding a linear unmixing matrix that minimizes this value. To accurately access dependency, the liner unmixing matrix is updated to minimize a dependency value while allowing a dependency evaluation network to perform self-supervised learning. This allows separation into a unique signal of each fluorescent molecule or protein.

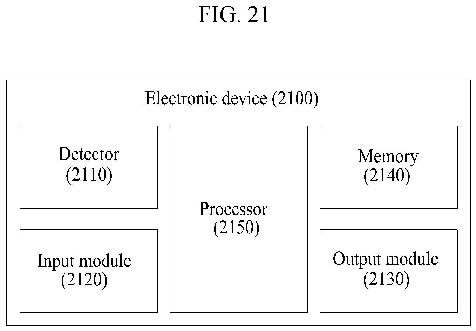

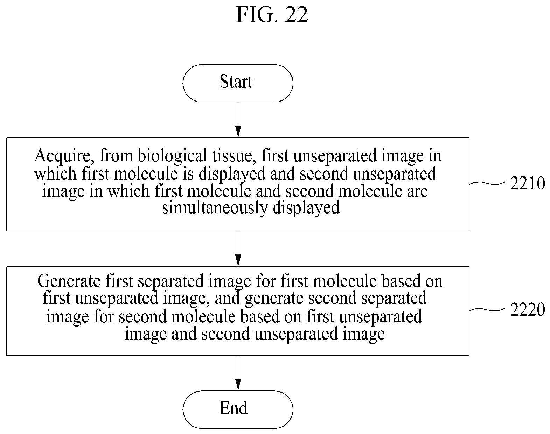

FIG. 21 is a block diagram illustrating an example of an internal configuration of an electronic device implemented as a computer device according to an example embodiment of the present invention, and FIG. 22 is a flowchart illustrating an example of an image processing method according to an example embodiment of the present invention.

Referring to FIG. 21, an electronic device 2100 according to various example embodiments of the present invention may include at least one of a detector 2110, an input module 2120, an output module 2130, a memory 2140, and a processor 2150. In some example embodiments, at least one of components of the electronic device 2100 may be omitted, or one or more other components may be added to the electronic device 2100.

The detector 2110 may capture an image of a specimen. Here, the detector 2110 may be installed at a predetermined location of the electronic device 2100 to capture the image. For example, the detector 2110 may include at least one of a scientific complementary metal-oxide-semiconductor (sCMOS) camera, a photo multiplier tube (PMT), and equipment that may measure intensity of light and expressing the same as an image.

The input module 2120 may receive an instruction or data to be used for at least one of the components of the electronic device 2100 from the outside of the electronic device 2100. Here, the input module 2120 may include at least one of an input device and a reception device. For example, the input device may include at least one of a microphone, a mouse, and a keyboard. In some example embodiments, the input device may include at least one of a touch circuitry configured to detect a touch and a sensor circuitry configured to measure the intensity of force generated by the touch. The reception device may include at least one of a wireless reception device and a wired reception device.

The output module 2130 may provide information to the outside of the electronic device 2100. Here, the output module 2130 may include at least one of a display device and a transmission device. For example, the display device may include at least one of a display, a holographic device, and a projector. **In** some example embodiments, the display device may be implemented as a touchscreen by being assembled to at least one of the touch circuitry and the sensor circuitry of the input module 2120. The transmission device may include at least one of a wireless transmission device and a wired transmission device.

According to an example embodiment, the reception device and the transmission device may be integrated into a single communication module. The communication module may support communication between the electronic device 2100 and an external device (not shown). This communication module may include at least one of a wireless communication module and a wired communication module. Here, the wireless communication module may include at least one of a wireless reception device and a wireless transmission device. The wireless communication module may support at least one of a far-distance communication scheme and a near-distance communication scheme. The near-distance communication scheme may include at least one of, for example, Bluetooth, WiFi direct, and infrared data association (IrDA). The wireless communication module may perform communication using the far-distance communication scheme through a network. The network may include, for example, a cellular network, the Internet, and a computer network such as a local area network (LAN) and a wide area network (WAN). Meanwhile, the wired communication module may include at least one of a wired reception device and a wired transmission device.

The memory 2140 may store at least one of a program and data used by at least one of the components of the electronic device 2100. For example, the memory 2140 may include at least one of a volatile memory and a nonvolatile memory. The memory 2140 may include two or more memories.

The processor 2150 may control at least one of the components of the electronic device 2100 by executing the program of the memory 2140, and may perform data processing or operations. The processor 2150 may include two or more processors.

Referring to FIG. 22, operations 2210 and 2220 of FIG. 22 may be performed by the electronic device 2100 that includes at least one processor (e.g., processor 2150). For example, the electronic device 2100 may perform each of operations 2210 and 2220 under control of the processor 2150 using instructions of a computer program stored in a recording medium such as the memory 2140.

**In** operation 2210, the electronic device 2100 may acquire, from a biological tissue, a first unseparated image in which a first molecule is displayed and a second unseparated image in which the first molecule and a second molecule are simultaneously displayed. For example, the electronic device 2100 may acquire the first unseparated image and the second unseparated image generated by irradiating light of different wavelengths to a specimen. **In** detail, for example, the electronic device 2100 may label the first molecule with a first material that contains a first fluorophore and label the first molecule and the second molecule with a second material that contains a second fluorophore and then, may acquire the first unseparated image that contains a signal of the first fluorophore and the second unseparated image that contains signals of the first fluorophore and the second fluorophore. Here, the first unseparated image may be acquired by detecting only light emitted from the first fluorophore, and the second unseparated image may be acquired by detecting only light emitted from the second fluorophore. Meanwhile, the first molecule may include a molecule within the biological tissue that is not labeled with a fluorophore and, in this case, the second molecule may be labeled with a second material that contains a second fluorophore. Also, the first unseparated image may contain a self-luminous signal of the biological tissue itself. Also, depending on example embodiments, the first unseparated image and the second unseparated image may be images acquired by irradiating light of the same wavelength to the specimen or may be images acquired by irradiating light of different wavelengths to the specimen. In each case, similarly, the first unseparated image may be an image acquired in a wavelength in which a signal of the second fluorophore is not detected.

In operation 2220, the electronic device 2100 may generate a first separated image for the first molecule based on the first unseparated image, and may generate a second separated image for the second molecule based on the first unseparated image and the second unseparated image. In an example embodiment, the electronic device 2100 may generate the first separated image and the second separated image using Gram-Schmidt (GS) orthogonalization according to the aforementioned ERASURE algorithm. In another example embodiment, the electronic device 2100 may calculate the first unseparated image and the second unseparated image using the unmixing matrix. Here, the unmixing matrix may be a matrix configured with variables calculated from all pairs of unseparated images. Here, in the aforementioned joint histogram-based signal separation technique, a value of at least one element included in the unmixing matrix may be used to define a loss function corresponding to the first unseparated image and the second unseparated image. Here, the loss function may be calculated using at least one of mutual information, Kullback-Leibler divergence, cross-entropy, and Rand-index. In this case, the electronic device 2100 may calculate variables that minimize the loss function, and may generate the first separated image and the second separated image using the variables. The joint histogram-based signal separation technique may refer to a technique disclosed in Korean Patent Application No. KR 10-2022-0096449, US17/931711. In still another example embodiment, a value of at least one element included in the unmixing matrix may be determined based on self-supervised learning. This technique utilizing self-supervised learning is based on the aforementioned self-supervised neural unmixing-based signal separation technique, and the self-supervised neural unmixing-based signal separation technique may refer to technologies disclosed in Korean Patent Application Nos. KR 10-2022-0109288, KR 10-2022-0150599, KR 10-2023-0052215, KR 10-2023-0113481, and PCT/KR2023/012865.

In another example embodiment, in operation 2210, the electronic device 2100 may label the first molecule with a first material that contains a first fluorophore, label the first molecule and the second molecule with a second material that contains a second fluorophore, and label the first molecule, the second molecule, and a third molecule with a third material that contains a third fluorophore and then, may acquire the first unseparated image that contains a signal of the first fluorophore, the second unseparated image that contains signals of the first fluorophore and the second fluorophore, and a third unseparated image that contains signals of the first fluorophore, the second fluorophore, and the third fluorophore. In this case, in operation 2220, the electronic device 2100 may generate the first separated image for the first molecule based on the first unseparated image, may generate the second separated image for the second molecule based on the first unseparated image and the second unseparated image, and may generate a third separated image for the third molecule based on the second unseparated image and the third unseparated image.

As described above, according to some example embodiments, there may be provided an image processing method and system for removing autofluorescence and cross-reactivity of antibodies using a signal separation algorithm.

The systems or the apparatuses described herein may be implemented using hardware components or a combination of hardware components and software components. For example, the apparatuses and the components described herein may be implemented using one or more general-purpose or special purpose computers, such as, for example, a processor, a controller, an arithmetic logic unit (ALU), a digital signal processor, a microcomputer, a field programmable gate array (FPGA), a programmable logic unit (PLU), a microprocessor, or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will be appreciated that a processing device may include multiple processing elements and/or multiple types of processing elements. For example, a processing device may include multiple processors or a processor and a controller. **In** addition, different processing configurations are possible, such as parallel processors.

The software may include a computer program, a piece of code, an instruction, or some combinations thereof, for independently or collectively instructing or configuring the processing device to operate as desired. Software and/or data may be permanently or temporarily embodied in any type of machine, component, physical equipment, virtual equipment, or a computer storage medium or device to be interpreted by the processing device or to provide an instruction or data to the processing device. The software also may be distributed over network coupled computer systems so that the software is stored and executed in a distributed fashion. The software and data may be stored by one or more computer readable storage media.

The methods according to example embodiments may be configured in a form of program instructions that may be performed through various computer methods and recorded in computer-readable media. The computer-readable media may include, alone or in combination with program instructions, data files and data structures. The media may continuously store computer-executable programs or may temporarily store the same for execution or download. Also, the media may be various types of recording devices or storage devices in a form in which one or a plurality of hardware components are combined. Without being limited to media directly connected to a computer system, the media may be distributed over the network. Examples of the media may include magnetic media such as hard disks, floppy disks, and magnetic tapes; optical media such as CD-ROM and DVDs; magneto-optical media such as floptical disks; and hardware devices that are specially configured to store and perform program instructions, such as ROM, RAM, flash memory, and the like. Also, examples of other media may include recording media and storage media managed by an app store that distributes applications or a site, a server, and the like that supplies and distributes other various types of software. Examples of the program instructions include an advanced language code executable by a computer using an interpreter, as well as a machine language code as produced by a compiler.

Although the example embodiments are described with reference to some specific example embodiments and accompanying drawings, it will be apparent to one of ordinary skill in the art that various alterations and modifications in form and details may be made in these example embodiments without departing from the spirit and scope of the claims and their equivalents. For example, suitable results may be achieved if the described techniques are performed in different order, and/or if components in a described system, architecture, device, or circuit are combined in a different manner, and/or replaced or supplemented by other components or their equivalents.

Therefore, other implementations, other example embodiments, and equivalents of the claims are to be construed as being included in the claims.

**Claims**

1.  An image processing method performed by a computer device comprising at least one processor and at least one memory in which instructions to be executed by the at least one processor are stored, the image processing method comprising:

    acquiring, from a biological tissue, a first unseparated image in which a first molecule is displayed and a second unseparated image in which the first molecule and a second molecule are simultaneously displayed; and generating a first separated image for the first molecule based on the first unseparated image, and generating a second separated image for the second molecule based on the first unseparated image and the second unseparated image.

2.  The image processing method of claim 1, wherein the acquiring comprises acquiring the first unseparated image and the second unseparated image generated by irradiating light of different wavelengths to a specimen.

3.  The image processing method of claim 1, wherein the generating comprises calculating the first unseparated image and the second unseparated image using an unmixing matrix.

4.  The image processing method of claim 3, wherein a value of at least one element included in the unmixing matrix is determined based on self-supervised learning.

5.  The image processing method of claim 3, wherein a value of at least one element included in the unmixing matrix is used to define a loss function corresponding to the first unseparated image and the second unseparated image, and the generating comprises:

    calculating variables that minimize the loss function; and generating the first separated image and the second separated image using the variables.

6.  The image processing method of claim 5, wherein the loss function is calculated using at least one of mutual information, Kullback-Leibler divergence, cross-entropy, and Rand-index.

7.  The image processing method of claim 1, wherein the generating comprises generating the first separated image and the second separated image using Gram-Schmidt (GS) orthogonalization.

8.  The image processing method of claim 1, wherein the acquiring comprises labeling the first molecule with a first material that contains a first fluorophore and labeling the first molecule and the second molecule with a second material that contains a second fluorophore and then, acquiring the first unseparated image that contains a signal of the first fluorophore and the second unseparated image that contains signals of the first fluorophore and the second fluorophore.

9.  The image processing method of claim 8, wherein the first unseparated image is acquired by detecting only light emitted from the first fluorophore, and the second unseparated image is acquired by detecting only light emitted from the second fluorophore.

10.  The image processing method of claim 8, wherein the first unseparated image and the second unseparated image contain amplified fluorescence signals.

11.  The image processing method of claim 1, wherein the acquiring comprises labeling the first molecule with a first material that contains a first fluorophore, labeling the first molecule and the second molecule with a second material that contains a second fluorophore, and labeling the first molecule, the second molecule, and a third molecule with a third material that contains a third fluorophore and then, acquiring the first unseparated image that contains a signal of the first fluorophore, the second unseparated image that contains signals of the first fluorophore and the second fluorophore, and a third unseparated image that contains signals of the first fluorophore, the second fluorophore, and the third fluorophore, and

the generating comprises generating the first separated image for the first molecule based on the first unseparated image, generating the second separated image for the second molecule based on the first unseparated image and the second unseparated image, and generating a third separated image for the third molecule based on the second unseparated image and the third unseparated image.

12. The image processing method of claim 1, wherein the first molecule includes a molecule within the biological tissue that is not labeled with a fluorophore, and
the second molecule is labeled with a second material that contains a second fluorophore.

13. The image processing method of claim 12, wherein the first unseparated image contains a self-luminous signal of the biological tissue itself.

14. The image processing method of claim 12, wherein the first unseparated image and the second unseparated image are images acquired by irradiating light of the same wavelength to a specimen.

15. The image processing method of claim 14, wherein the first unseparated image is an image acquired in a wavelength in which a signal of the second fluorophore is not detected.

16. The image processing method of claim 12, wherein the first unseparated image and the second unseparated image are images acquired by irradiating light of different wavelengths to a specimen.

17. The image processing method of claim 16, wherein the first unseparated image is an image acquired in a wavelength in which a signal of the second fluorophore is not detected.

18. A computer program stored in a computer-readable recording medium to execute an image processing method on a computer device, wherein the image processing method comprises:

acquiring, from a biological tissue, a first unseparated image in which a first molecule is displayed and a second unseparated image in which the first molecule and a second molecule are simultaneously displayed; and generating a first separated image for the first molecule based on the first unseparated image, and generating a second separated image for the second molecule based on the first unseparated image and the second unseparated image.

19. A computer device comprising:

at least one processor configured to execute computer-readable instructions on the computer device; and at least one memory configured to store the instructions to be executed by the at least one processor,
wherein the at least one processor causes the computer device to, acquire, from a biological tissue, a first unseparated image in which a first molecule is displayed and a second unseparated image in which the first molecule and a second molecule are simultaneously displayed, and
generate a first separated image for the first molecule based on the first unseparated image, and generate a second separated image for the second molecule based on the first unseparated image and the second unseparated image.

20. The computer device of claim 19, wherein, to acquire the first unseparated image and the second unseparated image, the at least one processor causes the computer device to acquire the first unseparated image and the second unseparated image generated by irradiating light of different wavelengths to a specimen.

FIG. 1

Anti-Rb secondary ab
(Alexa Fluor 488)

Anti-Rb secondary ab
(CF633)

The 2nd round
of staining

Rb anti-neuN ab

After the 1st
round of staining

Rb anti-GFAP abp

② ②

①

① = Type 1 cross-reactivity
② = Type 2 cross-reactivity

* Rb: Rabbit, ab: antibody

FIG. 2

FIG. 3

FIG. 4

EP 4 641 493 A1

FIG. 5

FIG. 6

FIG. 7

EP 4 641 493 A1

FIG. 8

EP 4 641 493 A1

FIG. 9

EP 4 641 493 A1

FIG. 10

FIG. 11

30 μm

EP 4 641 493 A1

FIG. 12

FIG. 13

FIG. 14

FIG. 15

FIG. 16

FIG. 17

FIG. 18

EP 4 641 493 A1

FIG. 19

FIG. 20

| H | | | | | | | The first step-round imaging |
| IMG1 | IMG2 | IMG3 | IMG4 | IMG5 | IMG6 | Overlay |

| I | The second step: Separation of signals from antibody cross-reactivity via ERASURE |
| IMG1 | IMG2 | IMG3 | IMG4 | IMG5 | IMG6 | Overlay |

| J | The third step: Spectral unmixing via PICASSO |
| ZNF3 | ATPB | Parvalbumin | NeuN | Calb2 | α-tubulin | Overlay |

FIG. 21

Electronic device (2100)

| Detector (2110) | Processor (2150) | Memory (2140) |
|---|---|---|
| Input module (2120) | | Output module (2130) |

FIG. 22

```
          ┌──────────────┐
          │    Start     │
          └──────────────┘
                 │
                 ▼
┌─────────────────────────────────────────────────┐
│ Acquire, from biological tissue, first           │
│ unseparated image in which first molecule is      │
│ displayed and second unseparated image in which   │      2210
│ first molecule and second molecule are            │
│ simultaneously displayed                          │
└─────────────────────────────────────────────────┘
                 │
                 ▼
┌─────────────────────────────────────────────────┐
│ Generate first separated image for first molecule │
│ based on first unseparated image, and generate    │
│ second separated image for second molecule based  │      2220
│ on first unseparated image and second unseparated │
│ image                                             │
└─────────────────────────────────────────────────┘
                 │
                 ▼
          ┌──────────────┐
          │     End      │
          └──────────────┘
```

## INTERNATIONAL SEARCH REPORT

| International application No. |
| --- |
| **PCT/KR2023/021440** |

### A. CLASSIFICATION OF SUBJECT MATTER

**G06T 7/11**(2017.01)i; **G06T 7/174**(2017.01)i; **G06T 7/00**(2006.01)i; **G06V 20/69**(2022.01)i; **G06N 3/08**(2006.01)i; **G01N 21/64**(2006.01)i

According to International Patent Classification (IPC) or to both national classification and IPC

### B. FIELDS SEARCHED

Minimum documentation searched (classification system followed by classification symbols)

G06T 7/11(2017.01); G01N 21/64(2006.01); G06K 9/00(2006.01); G06K 9/62(2006.01); G06N 20/00(2019.01); G06N 3/08(2006.01); G06T 7/13(2017.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 이미지(image), 분리(unmixing), 분자(molecule), 행렬(matrix), 파장(wavelength), 자발형광(autofluorescence)

### C. DOCUMENTS CONSIDERED TO BE RELEVANT

| Category* | Citation of document, with indication, where appropriate, of the relevant passages | Relevant to claim No. |
| --- | --- | --- |
| X | KR 10-2021-0087869 A (KOREA ADVANCED INSTITUTE OF SCIENCE AND TECHNOLOGY) 13 July 2021 (2021-07-13) See paragraphs [0013]-[0016], [0027]-[0031], [0052], [0079]-[0080], [0096]-[0097], [0124]-[0126], [0132]-[0133], [0235], [0280] and [0292]; and figures 2-6. | 1-3,5-20 |
| Y | | 4 |
| Y | US 2020-0226462 A1 (ROOM4 GROUP LIMITED) 16 July 2020 (2020-07-16) See paragraphs [0010]-[0016]. | 4 |
| A | KR 10-2022-0029358 A (KOREA ADVANCED INSTITUTE OF SCIENCE AND TECHNOLOGY) 08 March 2022 (2022-03-08) See paragraphs [0004]-[0006]; and claims 1-9. | 1-20 |
| A | KR 10-2020-0031687 A (FUJIFILM CORPORATION) 24 March 2020 (2020-03-24) See paragraphs [0008]-[0030]; and claims 1-7. | 1-20 |

✓ Further documents are listed in the continuation of Box C.    ✓ See patent family annex.

| | |
| --- | --- |
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **25 March 2024** | **28 March 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| | International application No. |
|---|---|
| | **PCT/KR2023/021440** |

| C. | DOCUMENTS CONSIDERED TO BE RELEVANT | |
|---|---|---|
| Category* | Citation of document, with indication, where appropriate, of the relevant passages | Relevant to claim No. |
| A | US 2020-0349340 A1 (VENTANA MEDICAL SYSTEMS, INC.) 05 November 2020 (2020-11-05) See paragraphs [0009]-[0048]; and claims 27-33. | 1-20 |

Form PCT/ISA/210 (second sheet) (July 2022)

## INTERNATIONAL SEARCH REPORT
### Information on patent family members

International application No.

**PCT/KR2023/021440**

| Patent document cited in search report | | | Publication date (day/month/year) | Patent family member(s) | | | Publication date (day/month/year) |
|---|---|---|---|---|---|---|---|
| KR | 10-2021-0087869 | A | 13 July 2021 | US | 11703454 | B2 | 18 July 2023 |
| | | | | US | 2021-0208076 | A1 | 08 July 2021 |
| US | 2020-0226462 | A1 | 16 July 2020 | CN | 110023994 | A | 16 July 2019 |
| | | | | CN | 110023994 | B | 30 October 2020 |
| | | | | EP | 3692497 | A1 | 12 August 2020 |
| | | | | EP | 3692497 | B1 | 03 November 2021 |
| | | | | GB | 2567155 | A | 10 April 2019 |
| | | | | GB | 2567155 | B | 02 March 2022 |
| | | | | US | 11232354 | B2 | 25 January 2022 |
| | | | | WO | 2019-068415 | A1 | 11 April 2019 |
| KR | 10-2022-0029358 | A | 08 March 2022 | EP | 3961194 | A1 | 02 March 2022 |
| | | | | EP | 3961194 | B1 | 08 November 2023 |
| | | | | KR | 10-2022-0026476 | A | 04 March 2022 |
| | | | | US | 2022-0065788 | A1 | 03 March 2022 |
| | | | | WO | 2022-045678 | A1 | 03 March 2022 |
| KR | 10-2020-0031687 | A | 24 March 2020 | EP | 3690017 | A1 | 05 August 2020 |
| | | | | JP | 7026694 | B2 | 28 February 2022 |
| | | | | US | 11257301 | B2 | 22 February 2022 |
| | | | | US | 2020-0184192 | A1 | 11 June 2020 |
| | | | | WO | 2019-065105 | A1 | 04 April 2019 |
| US | 2020-0349340 | A1 | 05 November 2020 | AU | 2016-313775 | A1 | 08 February 2018 |
| | | | | CA | 2994911 | A1 | 09 March 2017 |
| | | | | CA | 2994911 | C | 05 July 2022 |
| | | | | EP | 3345122 | A1 | 11 July 2018 |
| | | | | EP | 3345122 | B1 | 30 June 2021 |
| | | | | JP | 2018-529950 | A | 11 October 2018 |
| | | | | JP | 6725646 | B2 | 22 July 2020 |
| | | | | US | 10747986 | B2 | 18 August 2020 |
| | | | | US | 11361561 | B2 | 14 June 2022 |
| | | | | US | 2018-0204048 | A1 | 19 July 2018 |
| | | | | WO | 2017-037180 | A1 | 09 March 2017 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020200088091
- KR 1020210089419
- KR 1020210090057
- KR 1020210171431
- US 17132628 B
- US 17445629 B
- US 17567300 B
- KR 2021011097 W
- KR 2021018833 W

- EP 22150031
- EP 21192162
- KR 1020220096449
- US 17931711 B
- KR 1020220109288
- KR 1020220150599
- KR 1020230052215
- KR 1020230113481
- KR 2023012865 W